# EUROPEAN PATENT APPLICATION

(11) **EP 4 428 535 A1**
(43) Date of publication of application: **11.09.2024**
(21) Application number: 23382220.4
(22) Date of filing: 09.03.2023
(51) Int. Cl.: G01N 33/68

(54) **DIAGNOSIS, MONITORING OR PROGNOSIS OF DIABETES OR PREDIABETES**

(71) Applicant: Servizo Galego de Saude, 15703 Santiago de Compostela (ES); Fundación Instituto de Investigación Sanitaria de Santiago de Compostela (FIDIS), 15706 Santiago de Compostela (ES); Universidade de Santiago de Compostela, 15782 Santiago de Compostela (ES)
(72) Inventor: ALONSO SAMPEDRO, Manuela, 15706 Santiago de Compostela (ES); GONZÁLEZ QUÍNTELA, Arturo, 15782 Santiago de Compostela (ES); GUDE SAMPEDRO, Francisco, 15706 Santiago de Compostela (ES)
(74) Representative: Hoffmann Eitle

(57) **Abstract**

The present invention refers to an *in vitro* method for the diagnosis, monitoring or prognosis of diabetes or prediabetes; or for screening or selecting patient candidates to be subjected to OGTT, which comprises assessing the percentage of a protein, selected from the group consisting of: Retinol-Binding Protein 4 (Rbp), Alpha-1-acid glycoprotein (Agp) and/or fibronectin (FN), which is bound to glycosaminoglycans (GAGs).

## Description

### TECHNICAL FIELD

The present invention refers to the medical field. Particularly, the present invention refers to an *in vitro* method for the diagnosis, monitoring or prognosis of diabetes or prediabetes; or for screening or selecting patient candidates to be subjected to oral glucose tolerance test (OGTT), which comprises assessing the percentage of a protein, selected from the group consisting of: Retinol-Binding Protein 4 (Rbp), Alpha-1-acid glycoprotein (Agp) and/or fibronectin (FN), which is bound to glycosaminoglycans (GAGs).

### STATE OF THE ART

Diabetes mellitus is a complex, chronic illness characterized by elevated blood glucose levels that occurs when there is cellular resistance to insulin action, pancreatic β-cells do not produce sufficient insulin, or both. The number of cases and diabetes prevalence have greatly increased in recent decades; consequently, it is considered an important public health problem, one of four priority noncommunicable diseases (NCDs) targeted for action by world leaders.

Globally, an estimated 422 million adults were living with diabetes in 2014, compared to 108 million in 1980. The global prevalence (age-standardized) of diabetes has nearly doubled since 1980, rising from 4.7% to 8.5% in the adult population. The most common form of diabetes mellitus, type 2 diabetes (T2D), represents approximately 90% of all cases worldwide. T2D occurs more often in middle-aged and elderly adults, and its cause is multifactorial. However, its incidence has increased in children and young adults due to obesity, sedentary lifestyle, and inadequate nutrition. This high incidence is also accompanied by an estimate underdiagnoses prevalence of more than 50% worldwide.

Poor blood glucose control can result in long-term micro- and macrovascular complications such as nephropathy, retinopathy, neuropathy, and cardiovascular disease. Diabetes caused 1.5 million deaths in 2012. Higher-than-optimal blood glucose caused an additional 2.2 million deaths, by increasing the risks of cardiovascular and other diseases. Forty-three percent of these 3.7 million deaths occur before the age of 70 years.

Individuals with diabetes require continuous medical care, including pharmacological intervention as well as lifestyle and dietary changes. Diabetes and its complications bring about substantial economic loss to people with diabetes and their families and to health systems and national economies through direct medical costs and loss of work and wages.

According to the "Standards of Medical Care in Diabetes" published by the American Diabetes Association (ADA) and the World Health Organization (WHO) guidelines, diabetes may be diagnosed based on the concentration of plasma glucose -either fasting plasma glucose (FPG) or 2-hour plasma glucose during a 75g oral glucose tolerance test (OGTT)- or based on glycated hemoglobin A1c (A1C) concentration. Prediabetes is an intermediate hyperglycemic state in which glycemic markers such as blood glucose and A1C are above the threshold considered healthy but below the diagnostic criteria for diabetes. This state constitutes a high risk for the development of diabetes and complications associated with the loss of glycemic control. The diagnostic reference values for prediabetes and diabetes have not been universally standardized. However, the vast majority of clinical guidelines are based on the WHO and ADA criteria **(Table** 1). In the absence of unequivocal hyperglycemia, diagnosis requires two abnormal test results from the same sample or in two separate test samples. In a patient with classic symptoms of hyperglycemia or hyperglycemic crisis, the diagnosis is a random plasma glucose ≥ 200 mg/dL [American Diabetes Association. Standards of medical care in diabetes-2022. Diabetes Care, 2022; 45: S1. «Global Report on Diabetes». World Health Organization. 2016]*.*

**Table 1. Normoglycemia, Prediabetes and T2D diagnostic reference values**

| | **FPG** | **A1C** | **OGTT, 2-h PG** |
|---|---|---|---|
| Normoglycemia | | | NGT |
| ADA | < 100 mg/dL | < 5.7 % | < 140 mg/dL |
| WHO | < 110 mg/dL | | |

| Prediabetes | | | IGT |
|---|---|---|---|
| ADA | 100-125 mg/dL | 5.7-6.4% | 140-199 mg/dL |
| WHO | 110-125 mg/dL | | |

| Diabetes | | | |
|---|---|---|---|
| ADA | ≥ 126 mg/dL | ≥ 6.5% | ≥ 200 mg/dL |
| WHO | | | |

| | | | |
|---|---|---|---|
| *FPG, fasting plasma glucose (fasting is defined as no caloric intake for at least 8h); A1C, glycated hemoglobin A1c (the test should be performed in a laboratory using a method that is NGSP certified and standardized to the DCCT assay); OGTT, oral glucose tolerance test (the test should be performed using a glucose load containing the equivalent of 75g anhydrous glucose dissolved in water); 2-h PG, plasma glucose 2-h after OGTT; NGT, normal glucose tolerance; IGT, impaired glucose tolerance; ADA, American Diabetes Association; WHO, World Health Organization.* | | | |

It should be noted that the screening tests do not necessarily detect diabetes in the same individuals. The concordance between the FPG and 2-h PG test is imperfect, as is the concordance between A1C and either glucose-based test. Compared with FPG and A1C cut points, the 2-h PG value diagnoses more people with prediabetes and diabetes. In people in whom there is discordance between A1C values and glucose values, FPG and 2-h PG are more accurate.

Fasting plasma glucose has been the gold standard diagnostic criterion for T2D and is still the most widely accepted due to its availability, low cost, and compatibility with automated clinical chemistry analyzers. Among the disadvantages of FPG are that it requires at least 8h fasting, shows substantial biological and diurnal variability, reflects only a single point in time, and the samples involved present stability issues. Despite this, FPG is still widely used individually and as part of blood chemistry panels.

Glycated hemoglobin A1c is directly related to long-term average blood glucose levels and A1C level is strongly correlated with the development of complications due to hyperglycemia. A1C has several advantages compared with FPG and OGTT, including greater convenience (fasting not required), greater preanalytical stability, and less day-to-day perturbations during stress, changes in diet, or illness. However, these advantages may be offset by the lower sensitivity of A1C at the designated cut point, greater cost, and the imperfect correlation between A1C and average glucose in certain individuals. Moreover, age, race, ethnicity, and any clinical condition that alters the lifetime of erythrocytes or hemoglobin levels can alter A1C independent of glucose concentration. A1C testing using the ≥ 6.5% diagnostic threshold only diagnoses 30% of the total T2D cases identified through A1C, FPG, and OGTT collectively. Also, there is a low correlation between A1C and FPG, insulin resistance, and insulin secretion [Cowie CC, Rust KF, Byrd-Holt DD, Gregg EW, Ford ES, Geiss LS, Bainbridge KE, Fradkin JE. Prevalence of diabetes and high risk for diabetes using A1C criteria in the U.S. population in 1988-2006. Diabetes Care 2010; 33:562-568. doi: 10. 2337/dc09-1524]*.*

Oral glucose tolerance test is a marker of early impaired glucose homeostasis and is a more sensitive method of prediabetes and diabetes diagnosis than FPG and A1C. However, OGTT is relatively costly, can be complicated, and have low reproducibility in some settings. The test protocol requires that the patient ingest an oral load of 75g of glucose and undergo multiple blood draws over a two-hour period, which can be inconvenient and invasive for the patient. The need for timed samples creates logistical and analytical constraints. Despite its indication for T2D screening by the ADA, OGTT is not usually performed on non-pregnant adults. The OGTT performed between 24 and 28 weeks of gestation is the gold standard for the diagnosis of gestational diabetes (GD), with universal screening advised in populations with a high prevalence of T2D and obesity. In view of the cumbersome nature and poor reproducibility of the OGTT, it is necessary to look for and identify a more robust, convenient, and accurate biomarker for the diagnosis of GD.

So, in summary, there is an unmet clinical need to identify easily measurable and strong biomarkers, which are even more reliable than the traditional OGTT. The present invention is focused on solving this problem and a new and innovative strategy is herein provided for the diagnosis, monitoring or prognosis of diabetes or prediabetes; or for screening or selecting patient candidates to be subjected to OGTT.

### DESCRIPTION OF THE INVENTION

### Brief description of the invention

The present invention refers to an *in vitro* method for the diagnosis, monitoring or prognosis of diabetes or prediabetes; or for screening or selecting patient candidates to be subjected to OGTT, which comprises assessing the percentage of a protein, selected from the group consisting of: Rbp, Agp and/or FN, which is/are bound to GAGs. Kindly note that GAGs, also known as mucopolysaccharides, include heparan sulphate (HS), chondroitin sulphate (CS), dermatan sulphate (DS), keratan sulphate (KS), and hyaluronan (HA). Chemically, GAGs are diverse linear carbohydrate chains consisting of repeating units of disaccharides composed of a uronic acid moiety (d-glucuronic or l-iduronic acid) and an amino sugar (d-galactosamine or d-glucosamine) except for keratan sulphate, which contains repeating units of glucosamine and galactosamine. The amino sugars and uronic acid residues are often sulphated, except in the case of hyaluronan, which impart a high negative charge to the molecules and ensure linearity. Heterogeneity in the GAGs is due to differences in chain length, disaccharide content, disaccharide modifications, degree of sulfation, and charge density. Thus, GAGs are primarily large, heterogeneous, highly charged molecules. GAGs are components of endogenous large heterogeneous molecules known as the proteoglycans (PG). Proteoglycans consist of a protein core with varying numbers of GAGs attached. Proteoglycans are produced by all cells and can be found in the cell membranes, in secretory granules, or released into the surrounding extracellular environment [Saito A, Munakata H. Analysis of plasma proteins that bind to glycosaminoglycans. Biochim Biophys Acta 2007; 1770:241-246. doi: 10.1016/j.bbagen.2006.10.015] [Taylor KR, Gallo RL. Glycosaminoglycans and their proteoglycans: host-associated molecular patterns for initiation and modulation of inflammation. FASEB J. 2006; 20:9-22. doi: 10.1096/fj.05-4682rev]*.*

Particularly, the results provided by the inventors (see **Example 2** set below) show a predictive value of the proteins Rbp, Agp and/or FN bound to GAGs. So, they can be used for the diagnosis, monitoring or prognosis of diabetes or prediabetes; or for screening or selecting patient candidates to be subjected to OGTT. Interestingly, the results provided herein by the inventors show a predictive value of Rbp, Agp and/or FN bound to GAGs for inferring blood glucose levels 2 hours after ingestion of 75 g of glucose, i.e., after performing OGTT.

Therefore, according to the present invention, assessing the percentage of Rbp, Agp and/or FN bound to GAGs could be used in different scenarios:
- Quick and easy detection of diabetes/prediabetes.
- Prediction tool for the development of diabetes/prediabetes.
- Follow-up and monitoring of patients with diabetes/prediabetes.
- Reduction of the number of OGTT performed by two routes:
   o Screening of subjects with normal fasting glucose who may have diabetes/prediabetes and would be OGTT candidates.
   o Not having to repeat OGTT to confirm a diagnosis of diabetes/prediabetes.
- Substitution of OGTT by fasting levels of % Rbp, Agp and/or FN bound to GAGs.

Consequently, the first embodiment of the present invention refers to an *in vitro* method (hereinafter *first method of the invention*) for the diagnosis, monitoring or prognosis of diabetes or prediabetes which comprises assessing the percentage of a protein, selected from the group consisting of: Rbp, Agp and/or FN, which is/are bound to GAGs in a biological sample obtained from the subject, wherein the determination of a higher percentage of a protein, selected from the group consisting of: Rbp, Agp and/or FN, bound to GAGs, as compared with a pre-established threshold value, is an indication that the subject may be suffering from diabetes or prediabetes, or that the subject has a poor prognosis.

In a preferred embodiment, the method of the invention is characterized in that it is performed once an OGTT is carried out.

The second embodiment of the invention refers to an *in vitro* method (hereinafter *second method of the invention*) for screening or selecting patient candidates to be subjected to OGTT which comprises assessing the percentage of a protein, selected from the group consisting of: Rbp, Agp and/or FN, which is/are bound to GAGs in a biological sample obtained from the patient, wherein the determination of a higher percentage of a protein, selected from the group consisting of: Rbp, Agp and/or FN, which is bound to GAGs as compared with a pre-established threshold value, is an indication that the patient may be subjected to OGTT.

In a preferred embodiment, the above methods are performed in combination with the determination of fasting plasma glucose (FPG).

In a preferred embodiment, the biological samples are blood, plasma or serum.

The third embodiment of the present invention refers to the *in vitro* use of a protein selected from the group consisting of: Rbp, Agp and/or FN which is/are bound to GAGs, for the diagnosis, monitoring or prognosis of diabetes or prediabetes; or for screening or selecting patient candidates to be subjected to OGTT.

The fourth embodiment of the present invention refers to a kit consisting of tools and/or reagents for determining the percentage of a protein selected from the group consisting of: Rbp, Agp and/or FN which is/are bound to GAGs.

In a preferred embodiment the kit is an immunoassay which measures the presence or concentration of the above proteins bound to GAGs through the use of an antibody.

The fifth embodiment of the present invention refers to the use the above defined kit for the diagnosis, monitoring or prognosis of diabetes or prediabetes; or for screening or selecting patient candidates to be subjected to OGTT.

Alternatively, the present invention also refers to:
A method for detecting the percentage of a protein selected from the group consisting of: Rbp, Agp and/or FN which is/are bound to GAGs in a test sample from a human subject who may be suffering from diabetes or prediabetes, the method comprising the use of an immunoassay to detect and quantify the percentage of Rbp, Agp and/or FN which is/are bound to GAGs by, for instance:
a) Adding the biological sample to be analysed on the solid support,
b) Adding an antibody which may be conjugated with a detectable labelling agent,
c) Detecting and/or quantifying the signal obtained.

A method for analysing a biological sample prior to diagnosis, monitoring or prognosis of diabetes or prediabetes; or prior to screening or selecting patient candidates to be subjected to OGTT, comprising analysing the biological sample to assess the percentage of Rbp, Agp and/or FN which is/are bound to GAGs, wherein the determination of a higher percentage of a protein, selected from the group consisting of: Rbp, Agp and/or FN, bound to GAGs, as compared with a pre-established threshold value, is an indication that the subject may be suffering from diabetes or prediabetes, that the subject has a poor prognosis, or that the subject may be subjected to OGTT.

In a preferred embodiment, the present invention is a computer-implemented invention, wherein a processing unit (hardware) and a software are configured to:
- Receive the percentage of a protein, selected from the group consisting of: Rbp, Agp and/or FN, bound to GAGs,
- Process the percentage of a protein, selected from the group consisting of: Rbp, Agp and/or FN, bound to GAGs for finding substantial variations or deviations, and
- Provide an output through a terminal display of the percentage of a protein, selected from the group consisting of: Rbp, Agp and/or FN, bound to GAGs, wherein the determination of a higher percentage of a protein, selected from the group consisting of: Rbp, Agp and/or FN, bound to GAGs, as compared with a pre-established threshold value, is an indication that the subject may be suffering from diabetes or prediabetes, that the subject has a poor prognosis, or that the subject may be subjected to OGTT.

The last embodiment of the present invention refers to a method for treating patients suffering from diabetes or prediabetes, which comprises the administration of a therapeutically effective amount of treatment known in the prior art to treat diabetes, the method comprising performing, as a first step, the above first and or second methods of the invention.

Possible treatments to treat diabetes are depicted below:

| **Class** | **Compounds** |
|---|---|
| **Noninsulin glucose-lowering agents** | |
| Biguanides | Metformin |
| Sulfonylureas (2^{nd} generation) | Glimepiride, Glipizide, Glyburide |
| Thiazolidinediones | Pioglitazone, Rosiglitazone |
| α-Glucosidase inhibitors | Acarbose, Miglitol |
| Meglitinides (glinides) | Nateglinide, Repaglinide |
| DPP-4 inhibitors | Alogliptin, Saxagliptin, Linagliptin, Sitagliptin |
| SGLT2 inhibitors | Ertugliflozin, Dapagliflozin, Canagliflozin, Empagliflozin |
| GLP-1 RAs | Exenatide (extended release), Exenatide, Dulaglutide, Semaglutide, Liraglutide, Lixisenatide |
| Bile acid sequestrant | Colesevelam |
| Dopamine-2 agonist | Bromocriptine |
| Amylin mimetic | Pramlintide |

| **Insulins** | |
|---|---|
| Rapid-acting | Lispro follow-on product, Lispro, Lispro-aabc, Glulisine, Aspart, Aspart ("faster acting product"), Inhaled insulin |
| Short-acting | human regular |
| Intermediate-acting | human NPH |
| Concentrated human regular insulin | U-500 human regular insulin |
| Long-acting | Glargine follow-on products, Glargine, Detemir, Degludec |
| Premixed insulin products | NPH/regular 70/30, Lispro 50/50, Lispro 75/25, Aspart 70/30 |
| Premixed insulin/GLP-1 RA products | Glargine/Lixisenatide, Degludec/Liraglutide |

For the purpose of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of". Thus, the phrase "consisting of" indicates that the listed elements are required or mandatory, and that no other elements may be present.
- By "effective dose or amount" is intended an amount that, when administered as described herein, brings about a measurable response in the host. The exact amount required will vary from subject to subject, depending on the age, and general condition of the subject, the severity with which the disease has been/is progressed, mode of administration, and the like. An appropriate "effective" amount in any individual case may be determined by one of ordinary skill in the art using routine experimentation, based upon the information provided herein.
- According to the present invention, a reference value can be a "pre-established threshold value" or a "cut-off" value. Typically, a "threshold value" or "cut-off value" can be determined experimentally, empirically, or theoretically. According to the present invention, the "pre-established threshold" value refers to a value previously determined in subjects who are nonsuffering from diabetes or prediabetes. Thus, for instance, the subject is likely to suffer from diabetes or prediabetes, or to have a poor prognosis, when a higher percentage of a protein, selected from the group consisting of: Rbp, Agp and/or FN, bound to GAGs is identified, as compared with a pre-established "threshold value". A "threshold value" can also be arbitrarily selected based upon the existing experimental and/or clinical conditions, as would be recognized by a person of ordinary skilled in the art. The "threshold value" has to be determined in order to obtain the optimal sensitivity and specificity according to the function of the test and the benefit/risk balance (clinical consequences of false positive and false negative). Typically, the optimal sensitivity and specificity (and so the "threshold value") can be determined using a Receiver Operating Characteristic (ROC) curve based on experimental data.

### Description of the figures

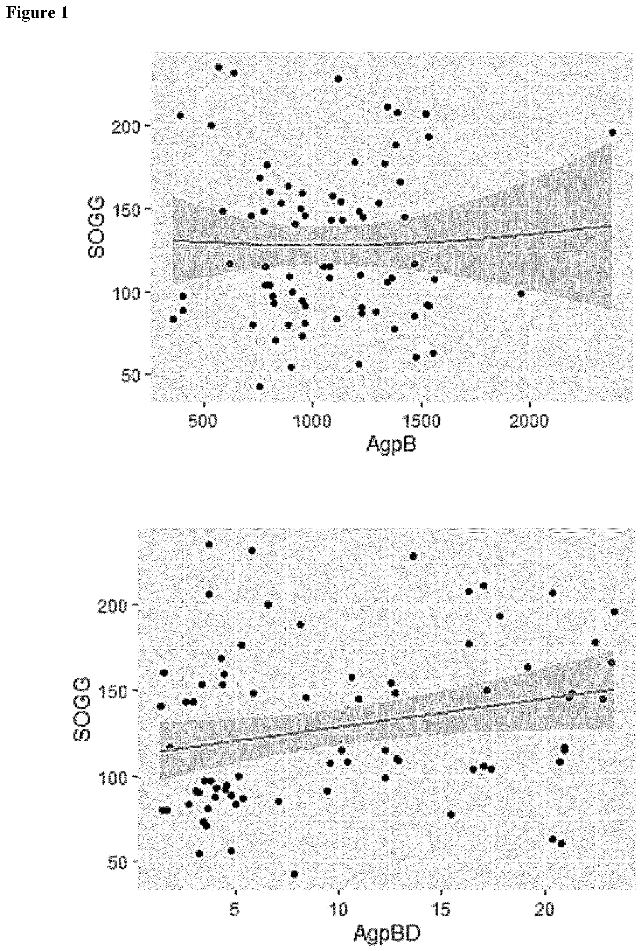

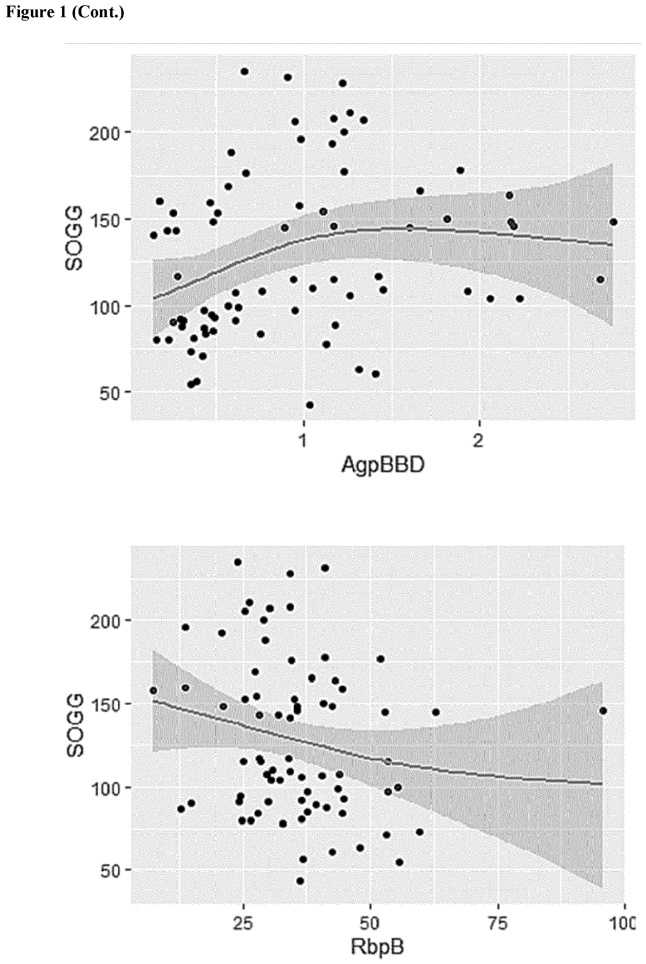

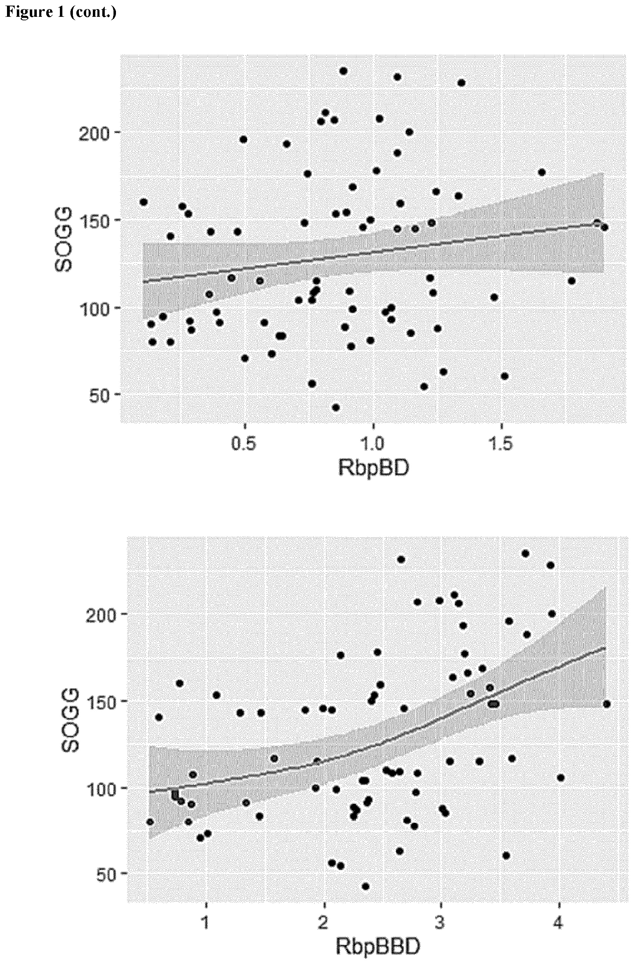

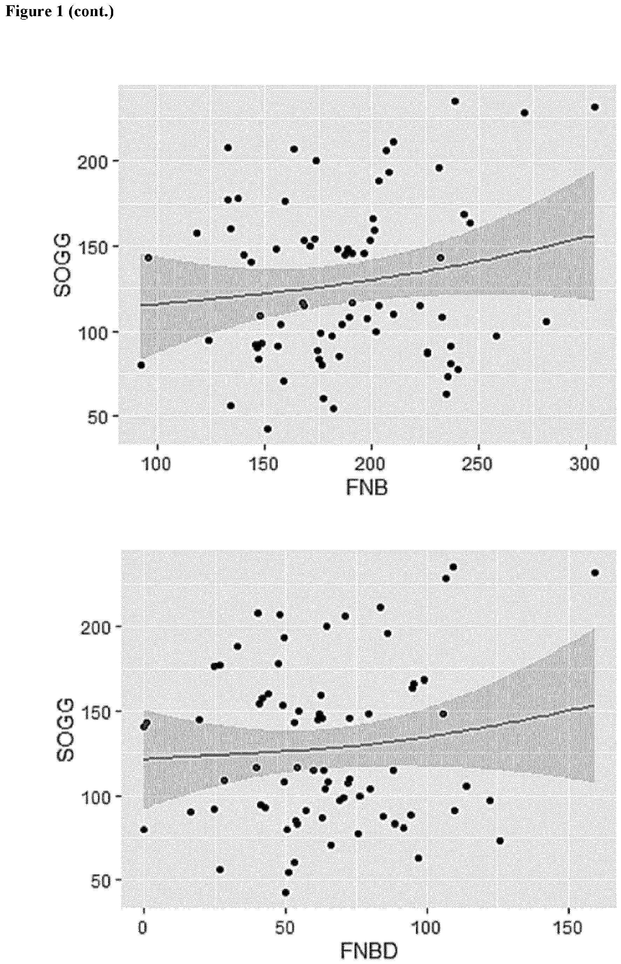

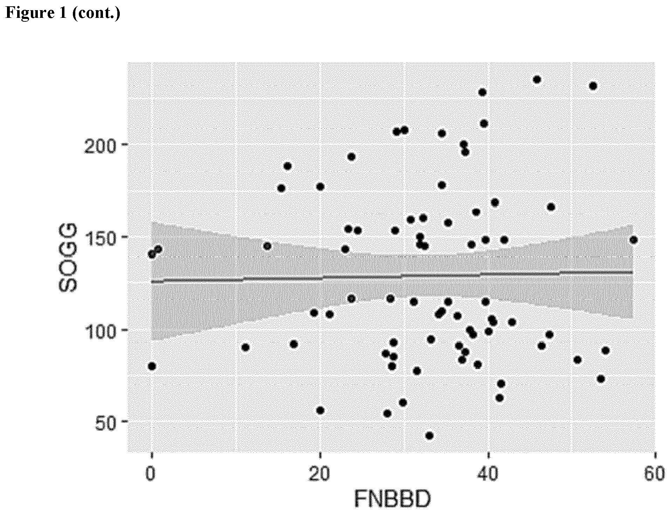

Figure 1. Relationship of basal serum levels of the three biomarkers studied with glucose levels at 2-h PG. GAG, glycosaminoglycans; RbpB, basal retinol-binding protein 4; RbpBD, basal retinol-binding protein 4 bound to GAG; % RbpBBD, percentage of basal RbpB bound to GAG; Agp, basal alpha-1-acid glycoprotein; AgpBD, basal alpha-1-acid glycoprotein bound to GAG; % AgpBBD, percentage of basal AgpB bound to GAG; FNB, basal fibronectin; FNBD, basal fibronectin bound to GAG; %FNBBD percentage of basal FNB bound to GAG; SOGG, serum glucose levels (mg/dL) 2 hours after an oral intake of 75 g of glucose.

### Detailed description of the invention

The present invention is illustrated by means of the Examples set below without the intention of limiting its scope of protection.

### Example 1. Material and Methods

### Example 1.1. Sample preparation

Blood samples were drawn in the morning after at least an 8 h overnight fast. Venous blood samples were collected using a serum separator tube (SST). Samples were allowed to clot for 30 minutes at room temperature before centrifugation for 15 minutes at 1000 g. Serum was collected and assayed immediately or was aliquot and store at -80°C. All participants underwent a standard 75 g OGTT with serum collected at 0 (fasting/basal) and 2 h.

### Example 1.2. Laboratory assays

Glucose was determined in plasma samples by the glucose oxidase peroxidase method on a fully automatic analyzed (ADVIA 2400 from Siemens Healthcare Diagnostics). HbA1c was determined by high-performance liquid chromatography on a Menarini Diagnostics HA-8160 analyzer. Insulin was determined by an immunochemometric assay on a Siemens ADVIA Centaur. All laboratory analyses were performed on the day of sample collection in the Clinical Biochemistry Laboratory of the University Hospital of Santiago de Compostela.

### Example 1.3. Assay procedure for separating the proteins bound to glycosaminoglycans

12.2 mg DMB (1.9-dimethylmethylene blue, Serva) was dissolved in 1 ml of ethanol 95%. Then, it was added to 99 ml of 0.2M sodium formiate buffer (formic acid + sodium formiate, pH 3.5). This DMB stock solution is stable in the dark for 30 days at room temperature. DMB stock solution was mixed 1:0.25 with serum (100 µl serum + 25 µl DMB solution) and vortex for 10 sec. The mixture was incubated at room temperature during 15 minutes for the formation of a precipitate, and centrifugated at 10.000 g x 10 min at 4°C. Carefully, the supernatant was removed by pipetting; and the precipitate containing proteins bound to sulfated GAG was resuspended in PBS (Phosphate Buffered Saline) [Alonso-Sampedro M, Álvarez-González V, Colón-Mejeras C, Garcia-González M, Lamas-González O. Method for separating the fraction bound to glycosaminoglycans and applications thereof. PCT WO 2017/042416 A1]*.*

### Example 1.4. Sample preparation of the GAG-bound proteins for subsequent studies (proteomics and ELISA)

All samples were prepared in duplicate and were concentrated 4x (100 µl of precipitate was resuspended by 25 µl PBS). Its quality was checked by determining the total protein concentration by Bradford assay (Bio-Rad). The coefficient of variation (CV) of the duplicates was calculated, and if it was higher than 30%, the sample was discarded, and the procedure was repeated. The samples of the GAG-bound proteins used in this study had on average a CV of 8.88% (0.21-28.64%). Once the quality was checked, the duplicates were joined in a single tube and stored at -20°C until use.

### Example 1.5. Proteomic studies using SWATH-MS (Sequential Window Acquisition of all Theoretical Mass Spectra)

The combination between SWATH-MS and DIA-MS (Data-independent Acquisition Mass Spectrometry) gives a protein quantification between different sample groups. Therefore, this method combines the targeted data extraction from DIA-MS files from previously identified proteins using higher-resolution mass analysis to perform a more accurate protein quantification.

The more abundant proteins from serum samples were removed adding DTT directly to 30 µl of serum at room temperature followed by removal of the precipitate via centrifugation. The supernatants were transferred to clean tubes. The GAG-bound proteins were used directly. Next, the samples were submitted to an in-gel concentration and in-gel digestion. From each sample, 4 µg of digested peptides was injected in a reversed-phase LC separation using the Nano-LC Ultra system Eksigent 425 in micro mode, coupled with the ekspert 400 autosampler (ABSciex). The eluted peptides are analyzed on a High-Resolution Quadrupole Time-of-Flight (QTOF instrument), TripleTOF 6600+ system (ABSciex). The pooled sample from each group was analyzed in the IDA mode to generate the spectral library. A DDA/IDA method was created using Analyst TF 1.7.1 with one survey scan and 60 candidate ion scans per cycle. The extraction of all the precursor information from these runs was done using software called PeakView that generates the entries needed for the Variable Window Calcutalor. With this tool, all the Q1 transmission windows needed were generated. The windows generated are exported to a text file that can finally be integrated into the Acquisition Control software (ABSciex). SWATH-MS data analysis was performed using PeakView 2.2 and SWATH-MS Microapp 2.0 (ABSciex). A detailed protocol can be found at Chantada-Vazquez et al, 2021. [Chantada-Vázquez MP, Garcia Vence M, Serna A, Núñez C, Bravo SB. (2021). SWATH-MS Protocols in Human Diseases. Methods in Molecular Biology (Vol. 2259, pp. 105-141). Humana Press Inc. https://doi.org/10.1007/978-1-0716-1178-4_7]

### Example 1.6. Determination of Retinol Binding Protein 4 levels

Levels of Rbp were determined using the #ab196264 Human RBP4 SimpleStep ELISA Kit from abcam. The lowest limit of detection was 2.6 pg/ml. The mean intra- and interassay CV were 5.1% and 8.9%, respectively. The assay was performed following the instructions of the manufacturer. Serum samples were diluted sequentially (1:50, 1:40, 1:40, and 1:5) using Sample Diluent NS (final dilution 1:400000). The GAG-bound proteins were diluted in PBS from 4x to 1x, and subsequently, they were diluted sequentially (1:50 and 1:40) using Sample Diluent NS (final dilution 1:2000). To perform the assay, samples or standards were added to the wells, followed by the antibody mix. After 1h of incubation at room t^{a}, the wells were washed to remove unbound material. TMB substrate was added and during incubation is catalyzed by HRP, generating blue coloration. This reaction was then stopped by addition of Stop Solution completing any color change from blue to yellow. Signal was generated proportionally to the amount of bound analyte and the intensity was measured at 450 nm. The concentration of Rbp was determined by interpolating blank control subtracted absorbance values against the standard curve. The resulting value was multiplied by the dilution factor to obtain the concentration in the sample. The measurement results in pg/ml were converted to µg/ml. All samples were assayed in duplicate, and the intraassay CV were 6.3% (0.38-21.92%) for serum Rbp and 4.7% (0.12-18.24%) for Rbp/GAG-bound.

### Example 1.7. Determination of Fibronectin levels

Levels of FN were determined using the #ab219046 Human Fibronectin SimpleStep ELISA Kit from abcam. The lowest limit of detection was 20.6 pg/ml. The mean intra- and interassay CV were 5.0% and 8.3%, respectively. The assay was performed following the instructions of the manufacturer. Serum samples were diluted sequentially (1:50, 1:40 and 1:20) using Sample Diluent NS (final dilution 1:40000). The GAG-bound proteins were diluted in PBS from 4x to 1x, and subsequently, they were diluted sequentially (1:50, 1:40 and 1:10) using Sample Diluent NS (final dilution 1:20000). To perform the assay, samples or standards were added to the wells, followed by the antibody mix. After 1h of incubation at room t^{a}, the wells were washed to remove unbound material. TMB substrate was added and during incubation is catalyzed by HRP, generating blue coloration. This reaction was then stopped by addition of Stop Solution completing any color change from blue to yellow. Signal was generated proportionally to the amount of bound analyte and the intensity was measured at 450nm. The concentration of FN was determined by interpolating blank control subtracted absorbance values against the standard curve. The resulting value was multiplied by the dilution factor to obtain the concentration in the sample. The measurement results in pg/ml were converted to µg/ml. All samples were assayed in duplicate, and the intrassay CV were 3.8% (0.15-16.31%) for serum FN and 3.3% (0.14-16.81%) for Rbp/GAG-bound.

### Example 1.8. Determination of Alpha 1 Acid Glycoprotein levels

Levels of Agp were determined using the #ab243675 Human alpha 1 Acid Glycoprotein SimpleStep ELISA Kit from abcam. The lowest limit of detection was 68.5 ng/ml. The mean intra- and interassay CV were 5.9% and 5.4%, respectively. The assay was performed following the instructions of the manufacturer. Serum samples were diluted sequentially (1:50 and 1:40) using 1x Wash Buffer PT, and then were further diluted sequentially (1:40 and 1:2) using Sample Diluent NS (final dilution 1:160000). The GAG-bound proteins were diluted in PBS from 4x to 1x, and subsequently, they were diluted 1:50 using 1x Wash Buffer PT, and then diluted 1:40 using Sample Diluent NS (final dilution 1:2000). To perform the assay, samples or standards were added to the wells, followed by the antibody mix. After 1h of incubation at room t^{a}, the wells were washed to remove unbound material. TMB substrate was added and during incubation is catalyzed by HRP, generating blue coloration. This reaction was then stopped by addition of Stop Solution completing any color change from blue to yellow. Signal was generated proportionally to the amount of bound analyte and the intensity was measured at 450nm. The concentration of Agp was determined by interpolating blank control subtracted absorbance values against the standard curve. The resulting value was multiplied by the dilution factor to obtain the concentration in the sample. The measurement results in ng/ml were converted to µg/ml. All samples were assayed in duplicate, and the intrassay CV were 4.9% (0.06-25.29%) for serum Agp and 4.6% (0.05-23.8%) for Agp/GAG-bound.

### Example 1.9. Statistical analyses

A descriptive analysis of the sample was conducted to obtain the mean and standard deviation for the quantitative variables, and the frequency and percentages for the qualitative variables. For the correlation studies, Spearman's test was used. To observe the differences between groups the one-way analysis of variance (ANOVA) test was used.

Logistic regression models were built to predict the probability of being a person with IGT and / or diabetes. Models were performed first entering only FPG or the basal levels of one of the biomarkers (total and bound to GAGs), and then, the combination of FPG and each of the biomarkers. Receiver operating curves were constructed to evaluate the discrimination power of the model and areas under the curve (AUC) were calculated.

Linear regression analysis with glucose levels after OGTT as the response variable were also performed to verify association with the biomarkers after adjusting by FPG. Only significant variables were included in the final model (p < 0.05).

All statistical analyses were carried out in R using the "rms" and "base" packages. These packages are freely available at cran.r-project.org.

### Example 1.10. Study population

The A Estrada Glycation and Inflammation Study (AEGIS I; trial NCT01796184 at www.clinicaltrials.gov) is an epidemiological study aimed to evaluate the relationship between inflammation and glycation factors and metabolic diseases, specifically diabetes and cardiovascular disease. Briefly, an age-stratified random sample of the population aged 18 years and older was drawn from Spain's National Health System Registry. There were 1516 subjects agreed to participate in the study (overall participation rate, 68%), made up of 678 men (45%) and 838 women (55%). From November 2012 through March 2015, all subjects were successively convened for one day at the Primary Care Center for evaluation, which comprised (1) an interviewer-administered structured questionnaire that included demographic and anthropometric data; (2) a lifestyle description, including diet, physical exercise, alcohol consumption, and smoking; and (3) fasting venous blood sampling and first void urine. In addition, 622 subjects consented to undergo a 6-day period of continuous glucose monitoring procedures. [Gude F, Diaz-Vidal P, Rúa-Pérez C, Alonso-Sampedro M, Fernández-Merino C, Rey-García J, Cadarso-Suárez C, Pazos-Couselo M, Garcia-López JM, González-Quintela A. Glycemic Variability and Its Association with Demographics and Lifestyles in a General Adult Population. J Diabetes Sci Technol. 2017; 11:780-790. doi: 10.1177/1932296816682031]*.*

The 5-years follow-up study (AEGIS II, PI16/01395) was ended in June 2020. The aim was to assess the efficacy of continuous glucose monitoring in predicting risk of type 2 diabetes, and to develop and validate a prediction risk model for type 2 diabetes after 5-year follow-up. The study comprises all the 1516 participants from the AEGIS I. After written informed consent, all the participants were re-evaluated for one day in the Primary Care Center. The standard workup includes a structured questionnaire, and clinical examination that allows for categorization of participants in terms of diabetes status according to the diagnostic criteria of the American Diabetes Association. Seven hundred and forty-six individuals agreed to participate (overall participation rate, 66.5%), 327 men (43.8%) and 419 women (56.2%). In addition, 236 subjects agreed to repeat the continuous glucose monitoring. The events of the incidence of diabetes and chronic diseases frequent in the population were monitored.

It was performed an OGTT on individuals not previously diagnosed with diabetes. The OGTT was used to screen diabetes and to provide a critical understanding of its future evolution. We enrolled 299 subjects, without prior diagnosis of diabetes, to undergo a 75g OGTT. According to the diagnostic criteria of the ADA **(Table 1),** the subjects were assigned to one of the three groups: i) normoglycemia/normal glucose tolerance (NGT); ii) prediabetes/impaired glucose tolerance; iii) T2D **(Table 2)**.

**Table 2. Classification according to the glycemic state of the individuals who performed the OGTT.**

| **Women** | **181 (60,5%)** | **Men** | **118 (39,5%)** |
|---|---|---|---|
| NGT | 166 (91,7%) | NGT | 97 (82,2 %) |
| Prediabetes/IGT | 12 (6,6%) | Prediabetes/IGT | 16 (13,6%) |
| T2D | 3 (1,7%) | T2D | 5 (4,2%) |

| | | | |
|---|---|---|---|
| *NGT, normoglycemia*/*normal glucose tolerance; IGT, impaired glucose tolerance; T2D, type 2 diabetes* | | | |

In both studies (AEGIS I and II), remaining blood and urine collected was freeze and conformed an ISCIII-collection (C.0004087).

### Example 2. Results

### Example 2.1. Proteomic pilot-study

Proteomics has been widely used in basic research and clinical diagnosis of human diseases; involves analysis, characterization, and classification of all protein in a sample. In particular, differential proteomics is a powerful technique based on comparing changes in protein levels between samples, which allows both to investigate the disease as a whole and to search for protein markers.

We conducted a proteomic pilot-study using the quantitative methodology SWATH-MS (Sequential Window Acquisition of all Theorical Mass Spectra). Xunta de Galicia (GPC-GAIN, IN607B 2018/1) funded the study. Of the 299 subjects who underwent the OGTT **(Table 2)**, we selected 15 men (60-70 years old): nine with normoglycemia/NGT, four with prediabetes/IGT and two with T2D. In the serum samples analyzed, we were able to measure the expression levels of 273 proteins. **Table 3** shows the differentially expressed proteins (p-value < 0.05 and >1.5-fold change up or down) between individuals classified as NGT (n=9) versus IGT/T2D (n=6).

### Example 2.2. Selection of the biomarkers to validate by ELISA

From the results of the proteomic pilot-study, we selected the most promising differentially expressed potential protein biomarkers (total or bound to glycosaminoglycans) with commercial ELISA available (funded by GRC-GAIN, IN607A 2021/ 1, Xunta de Galicia). The three selected proteins were retinol-binding protein 4 (Rbp), alpha-1-acid glycoprotein (Agp/ORM), and fibronectin (FN).

**Table 3. Potential proteins as biomarkers in diabetes from SWATH-MS proteomic analysis**

| **UniProt** # | **Protein** | **Gene** |
|---|---|---|
| P02753 | Retinol-binding protein 4 | RBP4 |
| P02763 | Alpha-1-acid glycoprotein 1 | ORM1/AGP1 |
| P02751 | Fibronectin | FN1 |
| P10909 | Clusterin | CLU (APOJ, CLI, KUB1) |
| P01701 | Immunoglobulin lambda variable 1-51 | IGLV1-51 |
| P18428 | Lipopolysaccharide-binding protein | LBP |
| P02765 | Alpha-2-HS-glycoprotein | AHSG (FETUA) |
| P00739 | Haptoglobin-related protein | HPR |
| P07737 | Profilin-1 | PFN1 |
| P00746 | Complement factor D | CFD (DF, PFD) |
| P24821 | Tenascin | TNC (HXB) |
| P35908 | Keratin, type II cytoskeletal 2 epidermal | KRT2 (KRT2A, KRT2E) |
| P01024 | Complement C3 | C3 (CPAMD1) |
| P01876 | Immunoglobulin heavy constant alpha 1 | IGHA1 |
| P01704 | Immunoglobulin lambda variable 2-14 | IGLV2-14 |
| P05546 | Heparin cofactor 2 | SERPIND1 (HCF2) |
| 014791 | Apolipoprotein L1 | APOL1 (APOL) |
| Q96KN2 | Beta-Ala-His dipeptidase | CNDP1 (CN1, CPGL2) |
| Q13093 | Platelet-activating factor acetylhydrolase | PLA2G7 (PAFAH) |
| P36955 | Pigment epithelium-derived factor | SERPINF 1 (PEDF) |

*Retinol-binding protein 4* is a member of the lipocalin family and the major transport protein of the hydrophobic molecule retinol, also known as vitamin A, in the circulation. Retinol binds to Rbp in the hepatocyte and after associating with transthyretin (TTR), the retinol/Rbp/TTR complex is released into the bloodstream and delivers retinol to tissues via binding to specific membrane receptors. Rbp is implicated in a variety of human conditions that include impaired vision and ocular diseases, disorders of glucose and lipid homeostasis, and cardiovascular diseases. [Steinhoff JS, Lass A, Schupp M. Biological functions of RBP4 and its relevance for human disease. Front Physiol 2021; 12:659977. doi: 10.3389/fphys.2021.659977. eCollection 2021].

*Alpha-1-acid glycoprotein* (also known as orosomucoid) is an abundant immunomodulatory protein in circulation, which is induced by stressful conditions such as infections and inflammation, and elevated levels have been found in diabetic patients. Expression of Agp has been linked with metabolic signaling, including hyperglycemia, and has been suggested to modulate immune responses to protect adipose tissue from inflammation and metabolic dysfunction. [Wurtz P, Tiainen M, Makinen VP, Kangas AJ, Soininen P, Saltevo J, Keinanen-Kiukaanniemi S, Mantyselka P, Lehtimaki T, Laakso M, Jula A, Kahonen M, Vanhala M, Ala-Korpela M. Circulating metabolite predictors of glycemia in middle-aged men and women. Diabetes Care 2012; 35: 1749-1756. doi: 10.2337/dc11-1838].

*Fibronectin* is a large dimeric glycoprotein that is expressed in various cell and tissue types and participates in multiple functions such as cell adhesion, growth, migration and differentiation. Cellular form of FN is synthetized by endothelial cells, fibroblasts and smooth muscle cells. FN is one of the most reliable proteins that can be estimated as a plasma indicator protein for endothelial function and related pathological disorders. Significant elevation of circulating FN has been reported in various metabolic syndromes associated with endothelial function, such as diabetes. The change in the levels of serum or plasma cellular FN may reflect the extent of matrix changes and vessel wall damage in patients with diabetes. Although various forms of FN have been implicated to have a role in metabolic disorders like diabetes the specific glycosylated version in question has only been investigated in just few studies. All forms of FN are glycosylated but, in these studies, the term glycosylated refers to specific sialyated glucose that SNA lectin recognizes. [Alanen J, Appelblom H, Korpimaki T, Kouru H, Sairanen M, Gissler M, Ryynanen M, Nevalainen J. Glycosilatedfibronectin as a first trimester marker for gestational diabetes. Arch Gynecol Obstet 2020, 302:853-860. doi: 10.1007/s00404-020-05670-8*].*

### Example 2.3. Validation of the biomarkers by ELISA

Of the 299 subjects who underwent the OGTT **(Table 2)**, we selected 74 case-control individuals based on sex, age, BMI (body mass index), and glycemic status at 2-h PG (normoglycemia/NGT, prediabetes/IGT, T2D). **Table 4** shows the characteristics of the 74 selected individuals, and as expected, as the glycemic status worsens, there was a significant increase in FPG, A1C and fructosamine.

**Table 5** shows the basal serum levels of the three selected biomarkers in individuals classified as normoglycemia/NGT, prediabetes/IGT, and type 2 diabetes. The levels of Rbp decrease (36.7 µg/ml vs 30.5 µg/ml) and those of Rbp bound to GAGs increase (0.79 µg/ml vs 0.99 µg/ml), without reaching statistical significance, as moved from normoglycemia to diabetes. The %Rbp bound to GAGs increase significantly from 2.14% to 3.28% (p = 0.004). No differences were observed in the levels of total Agp or in that bound to GAGs. In the levels of fibronectin bound to GAGs, a significant increase (65 µg/ml vs 85 µg/ml) was observed as moved from a state of normoglycemia to diabetes (p=0.034).

Our data show a non-significant decrease in Rbp in individuals with type 2 diabetes, however, in the study by Cho et al. found that the Rbp concentration increased significantly from the NGT group to the IGT and T2D groups (18.1, 18.9 and 20.9 µg/ml, respectively, p=0.002). They did not find significant differences between the IGT and NGT groups. Likewise, they found that the serum concentration of Rbp is associated with sex, men having a higher concentration than women (NGT 19.4 vs 16.2; IGT 22.5 vs 18.0; and DM 23.8 vs 20.1 µg/ml, p=0.049). In our study we also observed a significant increase in Rbp in men compared to women (NGT 39.4 vs 31.7; IGT 38.5 vs 32.6; and DM 30.7 vs 30.2 µg/ml, p=0.049). However, we did not find differences between sexes neither in the levels of Rbp bound to GAGs (p=0.279) nor in the % Rbp bound to GAGs (p=0.589). One possible cause of the discordant results is the different assays used in the measurement of Rbp. Graham et al. measured Rbp levels in subjects with insulin resistance and glucose intolerance and insulin-sensitive subjects with NGT using three commercial assays and a quantitative Western blot assay. They found substantial inconsistency between results with enzyme immunoassays that underestimated the Rbp levels and concluded that Western blot is the most reliable method to measure Rbp. However, we cannot rule out the possibility that this discrepancy was caused by the different measuring systems used (i.e., band intensity by Western blotting vs ELISA optical density) or by the different affinities of the antibodies used. [*Cho YM, Young BS, Lee H, Lee N, Min SS,* Kwak SH, Lee HK, Park KS. Plasma retinol-binding protein-4 concentrations are elevated in human subjects with impaired glucose tolerance and type 2 diabetes. Diabetes Care 2006, 29:2457-2461. doi: 10.2337/dc06-0360]*.* [Graham TE, Wason CJ, Blüher M, Kahn BB. Shortcomings in methodology complicate measurements of serum retinol binding protein (RBP4) in insulin-resistant human subjects. Diabetologia 2007, 50:814-823. doi: 10.1007/s00125-006-0557-0*].*

**Table 4. Clinical characteristics of the individuals analyzed**

| | NGT (n=40) | IGT (n=26) | T2D (n=8) | p value |
|---|---|---|---|---|
| Age, years | 59 ± 13 | 58 ± 14 | 61± 12 | 0.804 |
| Sex (W/M) | 14/26 | 11/15 | 3/5 | 0.698 |
| BMI, kg/m² | 31.1 ± 4.3 | 31.6 ± 6.0 | 33.1 ± 4.8 | 0.608 |
| **FPG, mg/dL^{[1,2]}** | 89 ± 9 | 97 ± 11 | 104 ± 11 | **0.000** |
| **A1C, % ^{[2,3]}** | 5.5 ± 0.3 | 5.6 ± 0.4 | 6.0 ± 0.5 | **0.003** |
| **Fructosamine, µmol/L** | 214 ± 31 | 230 ± 37 | 243 ± 48 | **0.043** |
| Insulin, mUI/L | 10.5 ± 6.1 | 14.9 ± 10.5 | 16.3 ± 12.5 | 0.064 |

| | | | | |
|---|---|---|---|---|
| NGT, normoglycemia/normal glucosa tolerance; IGT, prediabetes/impaired glucosa tolerance; T2D, type 2 diabetes; FPG, fasting plasma glucose; BMI, body mass index; A1C, glycosilated hemoglobin A1c; W, woman; M, man. Data are expressed as mean ± SD. The p-values were performed using ANOVA.1, Significant differences between NGT and IGT; 2, Significant differences between NGT and T2D; 3, Significant differences between IGT and T2D | | | | |

Hence, Rbp's causal role in inducing insulin resistance and T2D is still vividly debated within the field. The underlying reasons and whether or not Rbp is indeed actively contributing to insulin resistance is still under investigation. Are there yet unidentified post-translational modifications of Rbp that could mediate this? Our results show that the deterioration of glycemic status could be related to the binding of GAG to Rbp.

**Table 5. Basal serum levels of the three biomarkers according to glycemic status**

| | NGT (n=40) | IGT (n=26) | T2D (n=8) | p value |
|---|---|---|---|---|
| Rbp, µg/ml | 36.7 ± 10.9 | 36.0 ± 17.5 | 30.5 ± 5.8 | 0.486 |
| Rbp/GAG-bound, µg/ml | 0.79 ± 0.40 | 0.91 ± 0.48 | 0.99 ± 0.19 | 0.333 |
| **Rbp GAG-bound,% *** | 2.14 ± 0.90 | 2.58 ± 0.98 | 3.28 ± 0.51 | **0.004** |
| Agp, µg/ml | 1067 ± 358 | 1108 ± 361 | 938 ± 453 | 0.525 |
| Agp/GAG-bound, µg/ml | 8.82 ± 6.41 | 11.72 ± 7.83 | 10.90 ± 6.68 | 0.247 |
| Agp/GAG-bound, % | 0.84 ± 0.60 | 1.08 ± 0.74 | 1.09 ± 0.23 | 0.267 |
| FN, µg/ml | 187 ± 40 | 179 ± 39 | 212 ± 57 | 0.136 |
| **FN/GAG-bound, µg/ml *** | 65 ± 28 | 54 ± 28 | 85 ± 39 | **0.034** |
| FN/GAG-bound, % | 34 ± 11 | 29 ± 13 | 38 ± 8 | 0.107 |

| | | | | |
|---|---|---|---|---|
| *NGT, normoglycemia*/*normal glucose tolerance; IGT, prediabetes*/*impaired glucose tolerance; T2D, type 2 diabetes; Rbp, retinol-binding protein 4; Agp, alpha-1-acid glycoprotein; FN, fibronectin; GAG, glycosaminoglycans* *Data are expressed as mean* ± *SD. The p-values were performed using ANOVA.* ^{∗} *Significant differences between NGT and T2D* | | | | |

Glycosylated plasma FN has been indicated as an early predictor for gestational diabetes, however the studies have been contradictory. Rasanen et al (2013) found in their study a significant difference in glycosylated fibronectin concentrations between the GD group (132±36 mg/L, n=90) and the control group (80±4.0 mg/L, n=92, p<0.001). However, Alanen et al (2020) did not found difference in maternal serum glycosylated FN concentrations between women with consequent GD [447.5 µg/ml, interquartile range (IQR) 254.4-540.9 µg/ml, n=19] and control women (437.6 µg/ml, IQR 357.1-569.1 µg/ml, n=59). Maternal serum FN levels were significantly lower in GD group (224.2 µg/ml, IQR 156.8-270.6 µg/ml), compared to the control group (264.8 µg/ml, IQR 224.6-330.6 µg/ml, p<0.01). There is an ongoing prospective study using maternal serum glycosylated FN and/or OGTT 75g at 12-15 gestational weeks as a predictor of subsequent GD. Our data show a significant increase (65 µg/ml vs 85 µg/ml) in the levels of fibronectin bound to GAG as moved from a state of normoglycemia to diabetes (p=0.034). FN and %FN/GAG-bound concentrations were slightly higher in the T2D group compared to NGT (212±57 vs 187±40 µg/ml and 38±8 vs 34±11 µg/ml, respectively). [Rasanen JP, Snyder CK, Rao PV, Mihalache R, Heinonen S, Gravett MG, Roberts CT, Nagalla SR. Glycosylated fibronectin as a first-trimester biomarker for prediction of gestational diabetes. Obstet Gynecol 2013, 122: 586-94. doi: 10.1097/AOG.0b013e3182a0c88b]*,* [Alanen J, Appelblom H, Korpimaki T, Kouru H, Sairanen M, Gissler M, Ryynanen M, Nevalainen J. Glycosilatedfibronectin as a first trimester marker for gestational diabetes. Arch Gynecol Obstet 2020, 302:853-860. doi: 10.1007/s00404-020-05670-8]. [Huhn EA, Fischer T, Göbl CS, Bernasconi MT, Kreft M, Kunze M, Schoetzau A, Dolzlmuller E, Eppel W, Husslein P, Ochsenbein-Koelble N, Zimmermann R, Baz E, Prompeler H, Bruder E, Hahn S, Hoesli I. Screening of gestational diabetes mellitus in early pregnancy by oral glucose tolerance test and glycosylated fibronectin: study protocol for an international, prospective, multicentre cohort trial. BMJ Open 2016; 6: e012115. doi: 10.1136/bmjopen-2016-012115]*.*

Low-grade, systemic inflammation has been associated with the risk of diabetes. Prospective studies have demonstrated that higher Agp is associated with an increased risk of T2D. In the study of Wurtz et al (2012), Agp was prospectively associated with both fasting and postchallenge glucose. The results suggest that Agp is not only related to metabolic dysfunction cross-sectionally but also as a predictor for future glycemia, and thus highlight the role of prolonged inflammation as a risk marker for attenuating glucose tolerance. Tsuboi et al (2018) have recently demonstrated that serum Agp was related to postload glucose and glucose excursion during OGTT in non-obese young Japanese women. In our study, no differences were observed in the levels of total Agp or in that bound to GAG. [Wurtz P, Tiainen M, Makinen VP, Kangas AJ, Soininen P, Saltevo J, Keinanen-Kiukaanniemi S, Mantyselka P, Lehtimaki T, Laakso M, Jula A, Kahonen M, Vanhala M, Ala-Korpela M. Circulating metabolite predictors of glycemia in middle-aged men and women. Diabetes Care 2012; 35: 1749-1756. doi: 10.2337/dc11-1838]*.* [Tsuboi A, Minato S, Yano M, Takeuchi M, Kitaoka K, Kurata M, Yoshino G, Wu B, Kazumi T, Fukuo K. Association of serum orosomucoid with 30-min plasma glucose and glucose excursion during oral glucose tolerance tests in non-obese young Japanese women. BMJ Open Diabetes Res Care 2018; 6:e000508. doi: 10.1136/bmjdrc-2018-000508. eCollection 2018*].*

Next, we studied the relationship of the three protein biomarkers (total and bound to GAG) with glucose levels at 2-h PG as a continuous variable **(****Figure 1****)**. Agp bound to GAG levels increase as glucose increases, Agp levels do no change and the levels of the percentage of Agp bound to GAG first increase and then remain stable. The Rbp bound to GAG (total and in percentage) increases as glucose levels increase while Rbp levels decrease. No clear relationship was observed between glucose levels at 2-h PG and FN.

The levels of glucose at 2-h PG were positively correlated **(Table 6)** with FPG (0.452, p=0.000), A1C (0.391, p=0.001), insulin (0.301, p=0.009), % Rpb/GAG-bound (0.473, p=0.000), Agp/GAG- bound (0.243, p=0.037) and % Agp/GAG-bound (0.229, p=0.05). Serum Rbp concentration was negatively correlated with serum glucose levels at 2-h PG (-0.249, p=0.033). The association of the levels of glucose at 2-h PG with Rbp and Agp bound to GAG suggests an opportunity to discover insights into the mechanism of glucose dysmetabolism, although further research is needed.

It is known that proportions of individual GAG change during development and ageing processes. Thus, it appears that aging might affect the bound of GAG to proteins. In our study age was strongly correlated with Rbp, Agp and FN bound to GAG **(Table 7)**, and modestly correlated with Rbp (0.230, p=0.048).

**Table 6. Correlation between serum glucose levels at 2-h PG and various baseline parameters in the individuals analyzed (n=74).**

| | Spearman's Rho | p value |
|---|---|---|
| Age, years | 0.067 | 0.573 |
| BMI, kg/m² | 0.153 | 0.194 |
| **FPG, mg/dL** | **0.452** | **0.000** |
| **A1C, %** | **0.391** | **0.001** |
| Fructosamine, µmol/L | 0.221 | 0.059 |
| **Insulin, mUI/L** | **0.301** | **0.009** |
| **Rbp, µg/ml** | **-0.249** | **0.033** |
| Rbp/GAG-bound, µg/ml | 0.181 | 0.123 |
| **Rbp/GAG-bound, %** | **0.473** | **0.000** |
| Agp, µg/ml | 0.015 | 0.899 |
| **Agp/GAG-bound, µg/ml** | **0.243** | **0.037** |
| **Agp/GAG-bound, %** | **0.229** | **0.050** |
| FN, µg/ml | 0.103 | 0.383 |
| FN/GAG-bound, µg/ml | -0.001 | 0.992 |
| FN/GAG-bound, % | -0.003 | 0.982 |

| | | |
|---|---|---|
| *FPG, fasting plasma glucose; BMI, body mass index; A1C, glycosilated hemoglobin A1c; Rbp, retinol-binding protein 4; Agp, alpha-1-acid glycoprotein; FN, fibronectin; GAG, glycosaminoglycans; 2-h PG, serum glucose levels (mg*/*dL) 2 hours after an oral intake of 75 g of glucose.* | | |

**Table 7. Correlation between age and several parameters at baseline in the individuals analyzed (n=74).**

| | Spearman's Rho | p value |
|---|---|---|
| BMI, kg/m² | 0.212 | 0.070 |
| FPG, mg/dL | 0.102 | 0.388 |
| 2-h PG, mg/dL | 0.067 | 0.573 |
| A1C, % | 0.147 | 0.211 |
| Fructosamine, µmol/L | -0.006 | 0.957 |
| Insulin, mUI/L | 0.103 | 0.383 |
| **Rbp, µg/ml** | **0.230** | **0.048** |
| **Rbp/GAG-bound, µg/ml** | **0.317** | **0.006** |
| **Rbp/GAG-bound, %** | **0.265** | **0.023** |
| Agp, µg/ml | 0.139 | 0.237 |
| **Agp/GAG-bound, µg/ml** | **0.507** | **0.000** |
| **Agp/GAG-bound, %** | **0.494** | **0.000** |
| FN, µg/ml | 0.069 | 0.557 |
| FN/GAG-bound µg/ml | 0.212 | 0.070 |
| **FN/GAG-bound, %** | **0.253** | **0.030** |

| | | |
|---|---|---|
| *FPG, fasting plasma glucose; BMI, body mass index; A1C, glycosilated hemoglobin A1c; Rbp, retinol-binding protein 4; Agp, alpha-1-acid glycoprotein; FN, fibronectin; GAGs, glycosaminoglycans; 2-h PG, serum glucose levels (mg*/*dL) 2 hours after an oral intake of 75 g of glucose.* | | |

**Table 8** summarizes the correlation between the three protein biomarkers (total and bound to GAG). A strong positive significant relationship was observed between total protein (Rbp, Agp and FN) and protein-bound to GAG (0.606, p=0.000; 0.425, p=0.000; 0.780, p=0.000, respectively). Similarly, a strong positive significant relationship was observed between protein-bound to GAG and the percentage of protein-bound to GAGs for Rbp (0.642, p=0.000), Agp (0.888, p=0.000), and FN (0.894, p=0.000). There was a significant relationship between Rbp/GAG-bound, Agp/GAG-bound and %Agp/GAG-bound (0.577, p=0.000; 0.641, p=0.000, respectively). Likewise, there were a significant relationship between %Rbp/GAG-bound, Agp/GAG-bound and %Agp/GAG-bound (0.553, p=0.000; 0.598, p=0.000, respectively). No correlations were found between Rbp, Agp and FN protein levels; similarly, no correlations were found between Agp level and Rbp (total and bound to GAG), and FN level and Agp (total and bound to GAG). A significant inverse relationship was found between Agp and %FN/GAG-bound (-0.251, p=0.031).

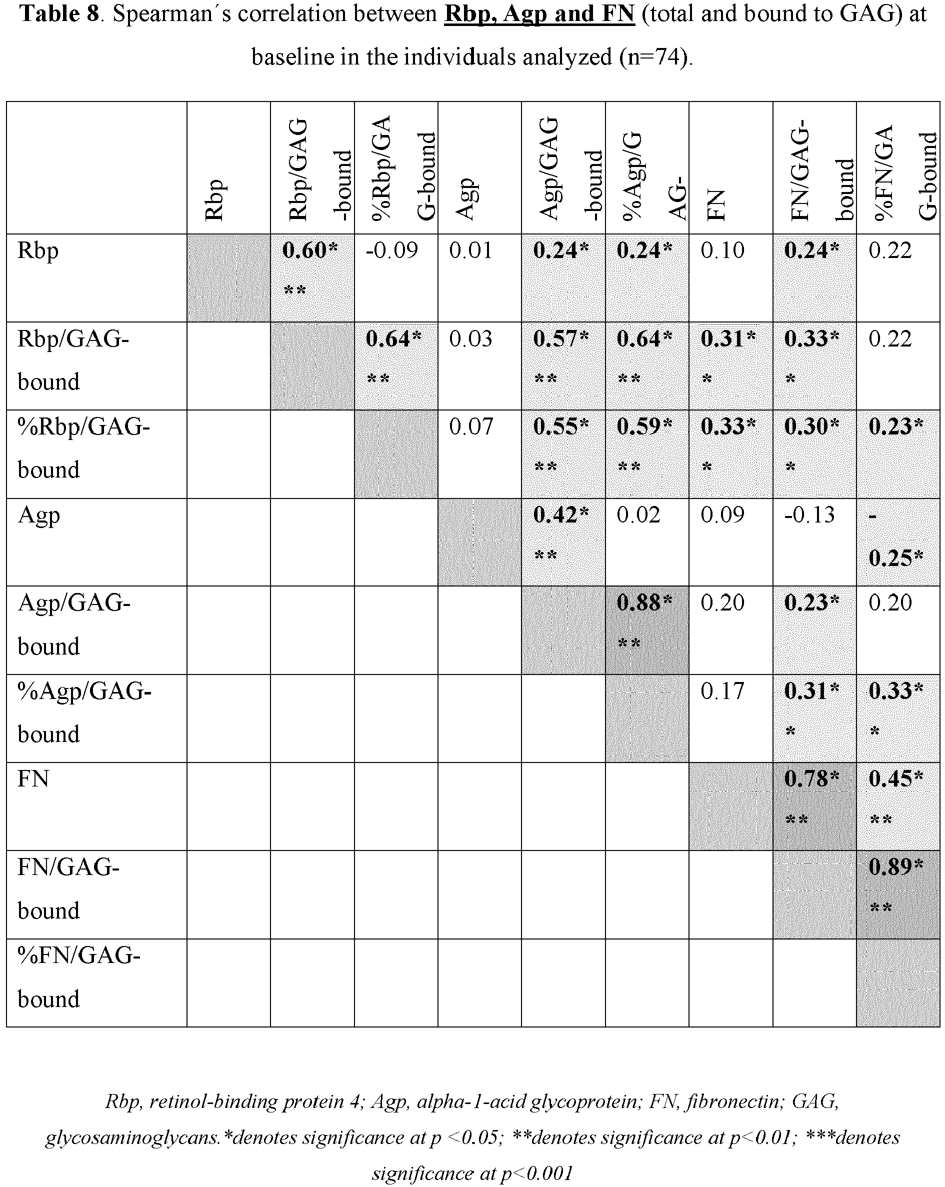

Our results show that the deterioration of glycemic status (measured by OGTT) is related to the binding of GAG to Rbp, Agp and FN proteins. However, they do not provide information about the type of GAG (HS, CS, DS, KS) to which they bind. These results may suggest a clue to identify novel pathways related to the glucose dysmetabolism. It is known that diabetes is accompanied by increased proteolytic in various tissues. This may lead to the cleavage of protein cores of proteoglycans releasing the GAG from these complexes. The products of proteoglycan degradation may penetrate into circulating blood and elevate the concentration of GAG in the serum. Thus, free GAGs could bind to the proteins present in the serum and cause a change in their function. This opens a new line of research on the effects of glucose on the binding of glycosaminoglycans to proteins with possible applications in the diagnosis, monitoring, prognosis and discovery of new therapeutic targets in prediabetes/IGT and /or diabetes.

### Example 2.4. Prediction of 2-h glucose levels after OGTT

We performed a stepwise logistic regression analysis including FPG, Rbp (total and bound to GAG), Agp (total and bound to GAG), and FN (total and bound to GAG). Because the number of individuals with values in the type 2 diabetes range is low (n=8), the predictive value of the combined Prediabetes/IGT+Type 2 diabetes (2-h PG > 139 mg/dL) and only Type 2 diabetes (2-h PG > 199 mg/dL) were studied separately **(Tables 9** and **Table 10,** respectively). In general, an increase in R² and discrimination capacity (AUC) was observed in the models that include FPG. The best performance observed in the prediabetes/IGT+Type 2 diabetes diagnostic model is FPG+%Rbp/GAG-bound (AUC 0.776). Adding the %Rbp/GAG-bound improves the predictive value of glucose levels at 2-h PG from FPG alone (AUC 0.736).

On the other hand, in the type 2 diabetes prediction model (2-h PG >199 mg/dL) the higher discrimination capacity (AUC 0.909) was observed when FPG+%Agp/GAG-bound was assayed.

Further, to identify the best predictive performance across the 2-h PG spectrum of glycemia after an OGTT, we tested different combinations of the three biomarkers (total and GAG-bound) and FPG using generalized additive models (GAM). The greatest R² (0.336) was observed when the response variable was the glucose levels at 2-h PG, and the predictors were FPG, Agp/GAG- bound and Rbp.

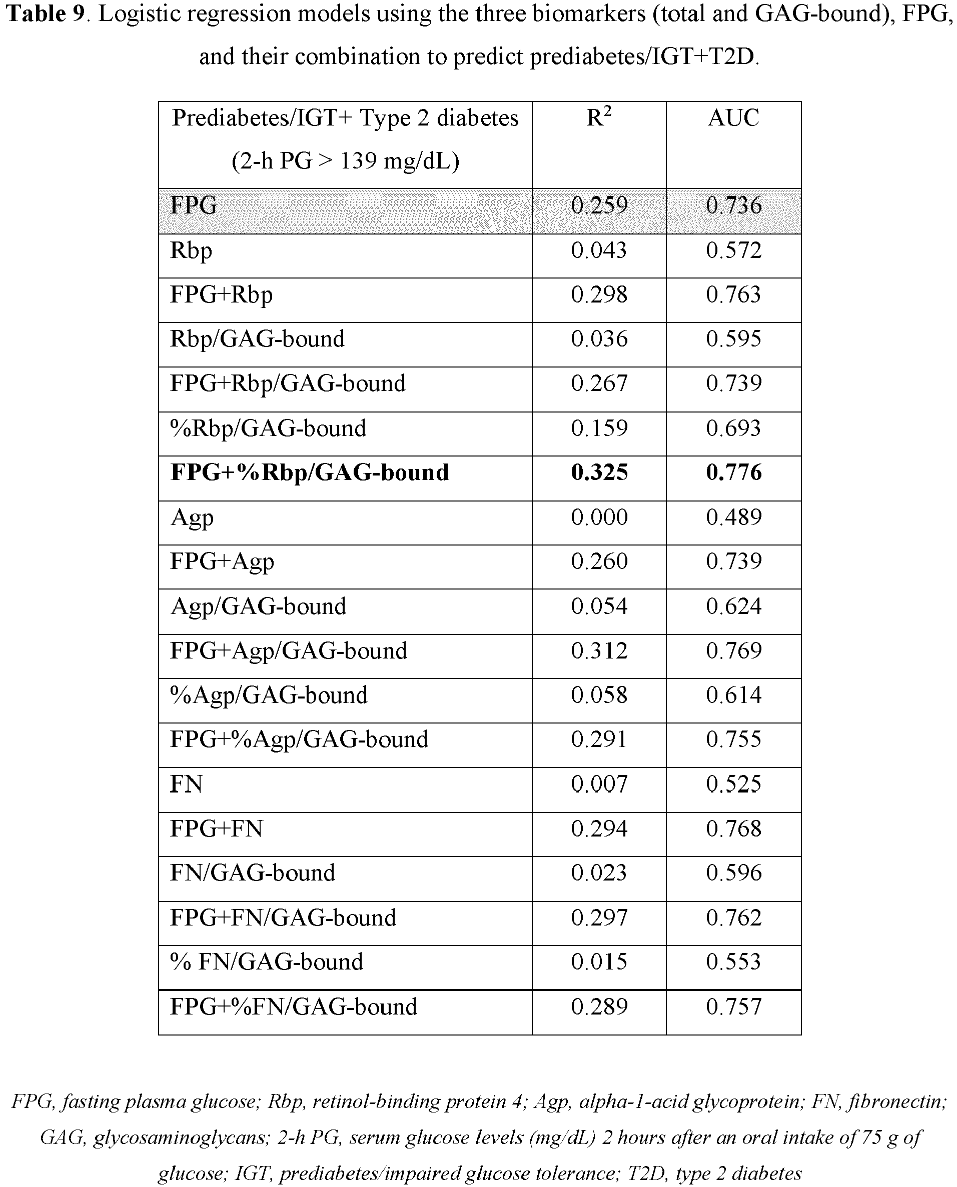

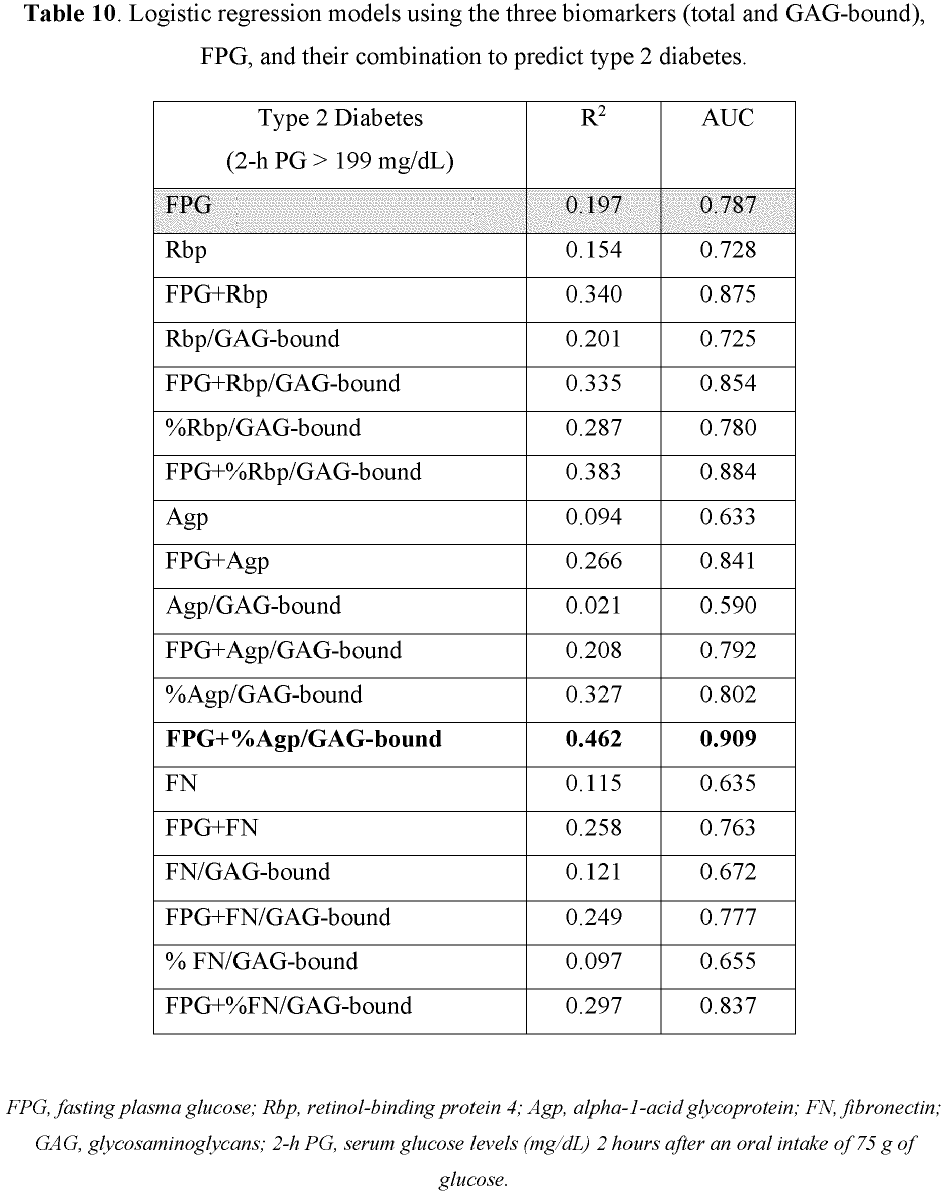

## Claims

1. *In vitro* method for the diagnosis, monitoring or prognosis of diabetes or prediabetes which comprises assessing the percentage of a protein, selected from the group consisting of: Retinol-Binding Protein 4, Alpha-1-acid glycoprotein and/or fibronectin which is/are bound to glycosaminoglycans, in a biological sample obtained from the subject, wherein the determination of a higher percentage of a protein, selected from the group consisting of: Retinol-Binding Protein 4, Alpha-1-acid glycoprotein and/or fibronectin bound to glycosaminoglycans, as compared with a pre-established threshold value, is an indication that the subject may be suffering from diabetes or prediabetes, or that the subject has a poor prognosis.

2. *In vitro* method, according to topic 1, **characterized in that** it is performed once an oral glucose tolerance test (OGTT) is carried out.

3. *In vitro* method for screening or selecting patient candidates to be subjected to OGTT which comprises assessing the percentage of a protein, selected from the group consisting of: Retinol-Binding Protein 4, Alpha-1-acid glycoprotein and/or fibronectin which is/are bound to glycosaminoglycans, in a biological sample obtained from the patient, wherein the determination of a higher percentage of a protein, selected from the group consisting of: Retinol-Binding Protein 4, Alpha-1-acid glycoprotein and/or fibronectin which is/are bound to glycosaminoglycans as compared with a pre-established threshold value, is an indication that the patient may be subjected to OGTT.

4. *In vitro* method, according to any of the claims 1 to 3, in combination with the determination of fasting plasma glucose (FPG).

5. *In vitro* method, according to any of the previous claims, wherein the biological samples is blood, plasma or serum.

6. *In vitro* use of a protein selected from the group consisting of: Retinol-Binding Protein 4, Alpha-1-acid glycoprotein and/or fibronectin which is/are bound to glycosaminoglycans, for the diagnosis, monitoring or prognosis of diabetes or prediabetes; or for screening or selecting patient candidates to be subjected to OGTT.

7. Immunoassay for determining the percentage of a protein selected from the group consisting of: Retinol-Binding Protein 4, Alpha-1-acid glycoprotein and/or fibronectin bound to GAGs.

8. Use of the immunoassay of claim 7 for the diagnosis, monitoring or prognosis of diabetes or prediabetes; or for screening or selecting patient candidates to be subjected to OGTT.
